# EUROPÄISCHE PATENTSCHRIFT

(11) **EP 2 521 525 B1**
(45) Veröffentlichungstag und Bekanntmachung des Hinweises auf die Patenterteilung: **05.08.2015**
(21) Anmeldenummer: 10725722.2
(22) Anmeldetag: 17.06.2010
(51) Int. Cl.: A61H 33/06, A61H 35/00, A61M 11/02, A61M 15/00, A61H 33/12, A61H 33/14

(54) **VERFAHREN UND VORRICHTUNG ZUR ERZEUGUNG EINES NANOAEROSOLS**
METHOD AND DEVICE FOR GENERATING A NANOAEROSOL
PROCÉDÉ ET DISPOSITIF POUR GÉNÉRER UN NANOAÉROSOL

(30) Priorität: 10.01.2010 EP 10150393
(43) Veröffentlichungstag der Anmeldung: 14.11.2012
(73) Patentinhaber: Medic Activ Vertriebs GmbH, 82031 Grünwald (DE); Zarfl, Hans Peter, 9400 Wolfsberg (AT)
(72) Erfinder: BRUNNER, Peter, 85737 Ismaning (DE); ZARFL, Hans Peter, A-9400 Wolfsberg (AT); LINDENA, Bernd Heiko, 82031 Grünwald (DE)
(74) Vertreter: Dilg, Haeusler, Schindelmann Patentanwaltsgesellschaft mbH
(86) Internationale Anmeldenummer: PCT/EP2010/058586
(87) Internationale Veröffentlichungsnummer: WO 2011/082838

(56) Entgegenhaltungen:
- EP-A2- 1 806 157
- US-A1- 2007 107 725
- US-A1- 2009 234 269

## Beschreibung

Die vorliegende Erfindung betrifft ein Verfahren und eine Vorrichtung zur Erzeugung eines Nanoaerosols durch Zerstäuben von Fluiden, insbesondere ein Verfahren und eine Vorrichtung zur Erzeugung eines Nanoaerosols, vor allem eines Nanoaerosols zu desinfizierenden Zwecken.

Es ist bekannt, dass flüssige Desinfektionsmittel als feinverteilte Tröpfchen versprüht werden können, um beispielsweise Gegenstände oder auch die mit schädlichen Aerosolen kontaminierte Raumluft zu desinfizieren. Seit langem ist außerdem die heilsame Wirkung von Dämpfen oder Aerosolnebeln, die aus feinverteilten Wasser- oder Öltröpfchen bestehen, bekannt. In herkömmlichen Sauna- oder Dampfbädern wird dazu Wasser oder eine wässrige Emulsion, die eine geringe Menge ätherischer Öle enthält, verdampft. Der heiße Dampf kühlt im Saunaraum ab und kondensiert zu Nebeltröpfchen. Ein Nachteil der Nebelerzeugung durch Verdampfen von Wasser oder einer wässrigen Ölemulsion liegt in der hohen Temperatur des erzeugten Nebels. Während die anregende Wirkung des heißen Nebels für bestimmte Anwendungen durchaus förderlich sein kann, sind herkömmliche Sauna- und Dampfbäder jedoch stets mit dem Nachteil verbunden, dass der Kreislauf der badenden Person hohen Belastungen ausgesetzt ist und daher Sauna- und Dampfbäder für große Bevölkerungsgruppen kontraindiziert sind. Es ist daher bereits vorgeschlagen worden, wässrige Lösungen oder Emulsionen bei niedrigeren Temperaturen, typischerweise bei Temperaturen von weniger als 35 oder 40°C, bevorzugt bei Temperaturen in einem Temperaturbereich von 22-28°C, zu zerstäuben. Damit wird der Kreislauf der zu behandelnden Personen im Vergleich zu herkömmlichen Sauna- oder Dampfbädern weniger belastet.

In der internationalen Patentanmeldung WO 00/44331 A1 wird eine Ganzkörper-Nebelbadvorrichtung und ein Verfahren zum Verabreichen eines Ganzkörper-Nebelbades beschrieben, mit welcher ein Nebelbad bei Temperaturen von weniger als 35°C erzeugt wird. Dazu wird eine Flüssigkeit auf einen Druck von mehr als 100 Bar komprimiert und anschließend über eine Düse explosionsartig in eine Behandlungskabine ausgestoßen, so dass die Flüssigkeit beim Ausstoßen in die Kabine infolge ihres hohen inneren Drucks in viele kleine Flüssigkeitströpfchen zerstäubt wird. Bereits in WO 00/44331 A1 ist vorgeschlagen worden, wässrige Salzlösungen oder Öle mit Vitaminzusätzen oder ätherischen Zusätzen zur Erzeugung eines Nebels zu verwenden. Wenn ein Nebel aus zwei unterschiedlichen Flüssigkeiten erzeugt werden soll, werden gemäß WO 00/44331 zwei separate Vernebler verwendet. Bei den in diesem Dokument beschriebenen Verneblern ist ein hohlzylinderförmiger Kompressionsraum mit einer verschließbaren Öffnung vorgesehen, der über eine Leitung mit einer Hochdruckpumpe verbunden ist, aus der die zu vernebelnde Flüssigkeit gefördert wird.

In der internationalen Patentanmeldung WO 2009/087053 A1 wird ein Verfahren und eine Vorrichtung zum Zerstäuben von Fluiden, insbesondere zur Erzeugung eines therapeutisch wirksamen Aerosols in einem Behandlungsraum beschrieben, wobei ein Aerosolerzeuger verwendet wird, der das zu zerstäubende Fluid mit Druck beaufschlagt und mit einer Fließgeschwindigkeit im Bereich von 50-300 m/s durch wenigstens eine Austrittsöffnung in Form von kleinen Partikeln in einen Behandlungsraum ausstößt. Bei einer Ausführungsform der in WO 2009/087053 beschriebenen Vorrichtung ist das zu zerstäubende Fluid in einer Kartusche angeordnet, welche wenigstens eine Austrittsöffnung zum Zerstäuben des Fluids und einen beweglichen Kolben aufweist, der durch eine Antriebseinrichtung bewegt werden kann. Mittels des beweglichen Kolbens wird ein solcher Druck auf das in der Kartusche befindliche Fluid ausgeübt, dass das Fluid mit der erforderlichen Fließgeschwindigkeit von 50-300 m/s durch die Austrittsöffnung in Form eines feinverteilten Flüssigkeitsnebels abgegeben wird.

In der europäischen Patentanmeldung EP1806157-A2 wird ein Verfahren und eine Kartusche zur erzeugung eines Nanoaerosols beschrieben.

Nachteilig an den oben beschriebenen bekannten Vorrichtungen ist unter anderem, dass Flüssigkeitsteilchen mit relativ breiter Größenverteilung erzeugt werden. Typischerweise enthält der Aerosolnebel ein Gemisch von Teilchen mit einem Durchmesser von wenigen Nanometern bis hin zu einigen Mikrometern. Bei zahlreichen Aerosolanwendungen sind aber insbesondere Teilchengrößen im Sub-Mikrometerbereich vorteilhaft und erwünscht. Werden in diesen Fällen auch Teilchen mit größerem Durchmesser abgegeben, so führt dies zu unnötigem Verlust von nicht wirksam eingesetztem Fluid, was insbesondere nachteilig ist, wenn es sich um ein wirkstoffhaltiges Fluid handelt.

Der vorliegenden Erfindung liegt daher das technische Problem zugrunde, ein Verfahren und eine Vorrichtung zur Erzeugung eines Nanoaerosols bereitzustellen, wobei im Wesentlichen Fluidpartikel mit einem Teilchendurchmesser von weniger als 1.000 Nanometer erzeugt werden. Die entsprechende Vorrichtung soll dabei einfach und kostengünstig herstellbar und betreibbar sein und insbesondere eine sterile Zerstäubung von wirkstoffhaltigen Fluiden ermöglichen.

Gelöst wird dieses technische Problem durch das Verfahren des vorliegenden Anspruchs 1, die Kartusche des vorliegenden Anspruchs 5 und den Aerosolerzeuger des vorliegenden Anspruchs 13.

Vorteilhafte Weiterbildungen des erfindungsgemäßen Verfahrens und der erfindungsgemäßen Vorrichtungen sind Gegenstände der abhängigen Patentansprüche.

Die Erfindung betrifft demnach ein Verfahren zur Erzeugung eines Nanoaerosols, wobei man in einer Düse wenigstens ein zu zerstäubendes Fluid durch eine Düsenöffnung der Düse entlang einer Austrittsöffnung in Form von Fluidpartikeln zerstäubt, die zerstäubten Fluidpartikel aus der ursprünglichen Austrittsrichtung ablenkt, und größere Fluidpartikel von kleineren Fluidpartikeln zumindest teilweise abtrennt, die abgetrennten größeren Fluidpartikel in das zu zerstäubende Fluid zurückführt und die kleineren Fluidpartikel an die Umgebung abgibt. Der Erfindung liegt die Überlegung zugrunde, dass beim Zerstäuben eines Fluids durch Ausstoßen des Fluids aus einer Düse zwangsläufig Fluidpartikel mit relativ breiter Größenverteilung entstehen. Statt die erzeugten Fluidpartikel unmittelbar an die Umgebung abzugeben, wird vorgeschlagen, die zerstäubten Fluidpartikel zunächst aus der Austrittsrichtung abzulenken, so dass es zu einer Abtrennung der größeren Fluidpartikel von den kleineren Fluidpartikeln kommen kann. Die schließlich an die Umgebung abgegebenen Fluidpartikel enthalten bei dem erfindungsgemäßen Verfahren einen deutlich reduzierten Anteil an größeren Partikeln, bevorzugt im Wesentlichen nur noch kleinere Fluidpartikel. Mit dem erfindungsgemäßen Verfahren können also kleinere Fluidpartikel in einem gewünschten Größenbereich erzeugt werden, während größere Fluidpartikel in das zu zerstäubende Fluid zurückgeführt werden, so dass ein Verlust an zu zerstäubendem Fluid durch Abgabe von Fluidpartikeln in einem unerwünschten Größenbereich vermieden und das zu zerstäubende Fluid im Wesentlichen vollständig in Form von Fluidpartikeln des gewünschten Größenbereichs zerstäubt werden kann. Der gewünschte Größenbereich der abgegebenen kleinen Fluidpartikel kann insbesondere durch Modifikation der rheologischen Eigenschaften des zu zerstäubenden Fluids und durch die Betriebsparameter und die Geometrie der Zerstäubungsvorrichtung eingestellt und optimiert werden.

Bei dem erfindungsgemäßen Verfahren verwendet man eine Kartusche, in welcher die Düse und das zu zerstäubende Fluid angeordnet sind. Bei der Kartusche, die weiter unten detaillierter beschrieben wird, kann es sich um eine Mehrwegkartusche handeln, besonders bevorzugt handelt es sich jedoch um eine Einwegkartusche.

Das Fluid kann auf unterschiedliche Weise aus der Düse ausgestoßen werden. Beispielsweise kann ein beweglicher Kolben vorgesehen sein, der Fluid zur Düsenöffnung presst. Im Gegensatz zu der beispielsweise in WO 2009/087053 A1 beschriebenen Kartusche würde der Kolben in einem solchen Fall das gesamte Fluid nicht in einer einzigen Hubbewegung ausstoßen, da im Rahmen der vorliegenden Erfindung vorgesehen ist, größere Fluidpartikel in das zu zerstäubende Fluid zurückzuführen. Vielmehr würde mit jeder Hubbewegung lediglich ein Teil des Fluids ausgestoßen werden, so dass mittels eines periodisch arbeitenden Kolbens über mehrere Zyklen das gesamte zu zerstäubende Fluid einschließlich der bei den jeweiligen Zyklen zurückgeführten größeren Partikel zerstäubt wird. Eine derartige Variante ist allerdings im Hinblick auf die Trennung von kleineren und größeren Fluidpartikeln nicht sehr effektiv, da ohne weitere Maßnahmen die kleineren Fluidpartikel rein passiv durch Diffusion an die Umgebung abgegeben werden. Außerdem ist ein mechanisch bewegter Kolben stets eine mögliche Fehlerquelle beim Betrieb der Vorrichtung.

Bei einer besonders bevorzugten Variante arbeitet man daher ohne beweglichen Kolben und erzeugt in der Düse einen Strom eines Trägergases und bringt das wenigstens eine zu zerstäubende Fluid in der Düse mit dem Trägergas in Kontakt. Das Fluid wird daher mittels des Trägergases aus der Düse ausgetragen und in die Fluidpartikel zerstäubt. Das Trägergas dient ferner dazu, die kleineren Fluidpartikel an die Umgebung abzugeben.

Die Düse ist dabei bevorzugt so ausgebildet, dass man das zu zerstäubende Fluid mittels eines in der Düse erzeugten Unterdrucks ansaugt und zerstäubt. Dieser Unterdruck kann durch das aus der Düse ausströmende Trägergas im Sinne des bekannten Venturi-Prinzips erzeugt werden.

Bei dem erfindungsgemäßen Verfahren verwendet man eine hermetisch verschlossene Kartusche, so dass sich das erfindungsgemäße Verfahren insbesondere zur Abgabe von steril in der Kartusche aufbewahrten, wirkstoffhaltigen Fluiden eignet. Vor Gebrauch bringt man dann Öffnungen in die Kartusche ein, welche die Zufuhr des Trägergases und die Abgabe der zerstäubten kleineren Fluidpartikel an die Umgebung ermöglichen. Je nach Einsatzzweck und verwendetem Fluid können aber auch verschließbare oder offene Kartuschen verwendet werden, die beispielsweise eine Wiederauffüllung im laufenden Betrieb ermöglichen, was bei hohem Fluidverbrauch wie beispielsweise in der Luftdesinfektion vorteilhaft sein kann.

Gemäß einer besonders einfachen Ausführungsform des erfindungsgemäßen Verfahrens werden die größeren Fluidpartikel unter Einwirkung der Schwerkraft in das zu zerstäubende Fluid zurückgeführt, während man die kleineren Fluidpartikel in einer von der Wirkungsrichtung der Schwerkraft verschiedenen Richtung, beispielsweise senkrecht nach oben, an die Umgebung abgibt. Dazu können beispielsweise im oberen Bereich der Kartusche Austrittsöffnungen vorgesehen sein, aus denen das Trägergas entweicht und die kleineren Fluidpartikel mitführt.

Das zu zerstäubende Fluid umfasst vorzugsweise zumindest einen Wirkstoff. Der Wirkstoff kann beispielsweise ausgewählt sein aus der Gruppe von Wirkstoffen bestehend aus Desinfektionsmitteln, desodorierenden Mitteln, Duftstoffen, kosmetischen Mitteln, diagnostischen und/oder therapeutischen Mitteln zur Behandlung von Lebewesen. Typische Wirkstoffe umfassen beispielsweise Arzneimittel, Hyaluronane, Vitamine, Säuerungsmittel oder pflanzliche und synthetische Öle. Ferne können auch das Immunsystem modulierende Substanzen, beispielsweise auch mit paramunisierenden Substanzen, als Wirkstoffe verwendet werden. Für die Luftdesinfektion geeignete Substanzen sind beispielsweise oxidative Substanzen wie Percarbonsäure. Das zu zerstäubende Fluid kann eine wässrige Lösung, eine wässrige Emulsion, insbesondere eine Öl-in-Wasser-Emulsion, eine ölige Lösung, eine ölige Emulsion, insbesondere eine Wasser-in-Öl-Emulsion, eine wässrige Suspension oder eine ölige Suspension sein. Dabei handelt es sich bei der wässrigen Lösung besonders bevorzugt um eine Salzlösung. Im Fall einer öligen Lösung handelt es sich besonders bevorzugt um eine Lösung, die ätherische Öle umfasst. Das zu zerstäubende Fluid kann außerdem spezielle Wirkstoffzubereitungen in Form von typischerweise negativ geladenen Lipidvesikeln umfassen, bei denen der Wirkstoff im Kern einer Lipidhülle oder in der Lipidhülle selbst befindet.

Mittel, die aus dem Wellness-Bereich bekannt sind, können verwendet werden.

Die zerstäubten kleineren Partikel, die an die Umgebung abgegeben werden, können beispielsweise mit einer stationären oder tragbaren Vorrichtung auf die zu behandelnden Gegenstände (beispielsweise im Fall eines Desinfektionsverfahrens) abgegeben werden. Besonders bevorzugt gibt man die zerstäubten kleineren Partikel jedoch in einen Behandlungsraum ab. Als Behandlungsraum kann dabei jeglicher Raum dienen, der abgestimmt auf den jeweiligen Anwendungsfall ermöglicht, dass beispielsweise die Haut oder die Atemwege eines zu behandelnden Menschen oder eines zu behandelnden Tieres mit den zerstäubten Nanoaerosolpartikeln in Kontakt kommen. Daher kann der Behandlungsraum beispielsweise auch eine Inhalationsmaske sein. Vorzugsweise ist der Behandlungsraum jedoch eine Behandlungskammer, welche das mit dem Nanoaerosol zu behandelnde Lebewesen ganz oder teilweise umgibt.

Bei einer bevorzugten Variante des erfindungsgemäßen Verfahrens lädt man zumindest einen Teil der abzugebenden kleineren Fluidpartikel elektrostatisch auf. Wenn man die zu behandelnden Objekte oder Lebewesen erdet oder mit einem entsprechend entgegengesetzten Potential verbindet, kann eine besonders effektive Behandlung erzielt werden, da dann die abgegebenen kleineren Fluidpartikel von dem zu behandelnden Objekt oder Lebewesen angezogen werden.

Erfindungsgemäß weisen die kleineren Fluidpartikel einen Durchmesser von weniger als 1.000 Nanometer, vorzugsweise einen Durchmesser von 5-750 nm, insbesondere von 5-300 nm, und besonders bevorzugt einen Durchmesser von 10-300 nm, insbesondere von 10-200 nm auf. Durch Anpassung der rheologischen Eigenschaften des zu zerstäubenden Fluid, durch Optimierung der Düse sowie des Drucks und/oder der Durchflussmenge des Trägergases lässt sich der mittlere Durchmesser der ausgetragenen kleineren Fluidpartikel in gewissen Grenzen variieren. Da in dem abgegebenen Aerosolnebel praktisch keine Fluidpartikel mit einem Durchmesser von mehr als einem Mikrometer enthalten sind, sind beispielsweise bei Inhalationsanwendungen praktisch alle erzeugten Partikel sehr gut lungengängig. Bei Desinfektionsanwendungen wird bei Verwendung eines Nanoaerosols kein Feuchtigkeitsfilm auf den zu desinfizierenden Objekten abgelagert, so dass gewissermaßen eine "trockene" Desinfektion möglich ist. Schädliche Erreger, die in schwebenden Tröpfchen vorhanden sind, können durch Kollision mit den erfindungsgemäß erzeugten Fluidpartikeln bekämpft werden, die beispielsweise eine Erniedrigung des pH-Wertes in oder Oxidationen in den erregerhaltigen Tröpfchen hervorrufen.

Die Erfindung betrifft außerdem eine Kartusche zur Erzeugung eines Nanoaerosols, mit wenigstens einem Reservoir für das zu zerstäubende Fluid, einer Entmischungskammer, die eine Ablenkeinrichtung und wenigstens eine Austrittsöffnung für das zu zerstäubende Fluid aufweist, einer Düse, die wenigstens eine in die Entmischungskammer mündende Düsenöffnung aufweist und wenigstens einen von dem Reservoir ausgehenden Kanal, der in die Düse mündet. Die Düsenöffnung befindet sich vorzugsweise auf einer Längsachse der Düse, in deren Verlängerung die Ablenkeinrichtung angeordnet ist.

Vorzugsweise besteht eine kommunizierende Verbindung zwischen dem Reservoir und der Entmischungskammer, so dass ein Teil der abgelenkten Fluidpartikel, insbesondere Fluidpartikel mit größerem Durchmesser, aus der Entmischungskammer in das Reservoir zurückgelangen können.

Wie oben erwähnt, arbeitet die Düse bei einer besonders bevorzugten Ausführungsform mit einem Trägergas. Daher ist bevorzugt wenigstens eine in die Düse mündende Zuleitung für ein Trägergas vorgesehen.

Bei einer Variante der erfindungsgemäßen Kartusche ist die Zuleitung für das Trägergas als zentrale Bohrung ausgebildet, die an einem Ende in den Boden der Kartusche mündet und an ihrem anderen Ende in die Düse übergeht.

Zur Bildung einer hermetisch verschlossenen Kartusche ist die in den Boden der Kartusche mündende Bohrung durch eine durchstoßbare Wand verschlossen. In diesem Fall ist außerdem vorzugsweise die wenigstens eine Austrittsöffnung für das zu zerstäubende Fluid durch eine durchstoßbare oder aufreißbare Wand verschlossen. Beispielsweise können auf der Oberseite der Kartusche eine oder mehrere Aufreißlaschen angeordnet sein, mit deren Hilfe Öffnungen in der Oberseite erzeugt werden können.

Gemäß einer bevorzugten Variante der erfindungsgemäßen Kartusche ist die Düse als Venturi-Düse ausgebildet.

Die Kartusche kann außerdem Mittel zur Verbindung des Reservoirs mit einer elektrischen Spannungsquelle aufweisen. Bei einer Variante, bei welcher die Kartusche ausschließlich aus einem elektrisch nichtleitenden Material, beispielsweise aus einem nichtleitenden Kunststoffmaterial besteht, können diese Mittel beispielsweise aus einer durchstoßbaren, zunächst verschlossenen Öffnung im Boden der Kartusche bestehen, durch welche vor Gebrauch der Kartusche eine mit einer Spannungsquelle verbundene Elektrode so in die Kartusche eingeführt wird, dass die Elektrode mit dem zu zerstäubenden Fluid in Kontakt kommt. Bei einer anderen Variante kann die Düse zumindest teilweise aus einem elektrisch leitfähigen Material bestehen und es können Mittel zum Verbinden der Düse mit einer elektrischen Spannungsquelle vorgesehen sein.

Gemäß einer bevorzugten Ausführungsform besteht die Kartusche aus zumindest zwei Bauteilen, die nach Einfüllen des zu zerstäubenden Fluids miteinander verbindbar sind. Bei der Verbindung der beiden Bauteile kann es sich beispielsweise um eine Steckverbindung, eine Schraubverbindung oder Ähnliches handeln. Besonders bevorzugt ist die Verbindung so ausgebildet, dass nach Einfüllen des zu zerstäubenden Fluids eine vom Benutzer nicht mehr lösbare Verbindung der beiden Bauteile hergestellt wird. Beispielsweise können die beiden Bauteile miteinander verklebt oder verschweißt werden. Das zu zerstäubende Fluid wird hermetisch in der Kartusche verschossen, so dass bis zum Gebrauch der Kartusche gewährleistet werden kann, dass keine Verschmutzung von außen eindringt und beispielsweise Sterilität des enthaltenen Fluids gewährleistet werden kann.

In der so verschlossenen Kartusche befindet sich dann das zu zerstäubende Fluid.

Gegenstand der Erfindung ist außerdem ein Aerosolerzeuger, der eine Halterung zur Aufnahme wenigstens einer Kartusche der oben beschriebenen Art und Mittel zum Ausstoßen des in der Kartusche enthaltenen Fluids in Form eines Nanoaerosols umfasst.

Vorzugsweise weisen die Mittel zum Ausstoßen des in der Kartusche enthaltenen Fluids eine Quelle eines Trägergases auf, wobei die Halterung wenigstens einen ersten hohlen Dorn, der mit der Quelle eines Trägergases kommuniziert und in die Zuleitung für das Trägergas der Kartusche eingreifen kann, und wenigstens einen zweiten hohlen Dorn, der mit der Umgebung kommuniziert und in die Austrittsöffnung für das zu zerstäubende Fluid der Kartusche eingreifen kann, umfasst. Vor dem Ausstoßen des Fluids werden der erste hohle Dorn und der zweite hohle Dorn mechanisch, beispielsweise hydraulisch oder pneumatisch in die hermetisch verschlossene Kartusche eingetrieben. Anstelle des zweiten hohen Dorns zum Öffnen der Austrittsöffnungen der Kartusche kann die Kartusche selbst mit geeigneten Aufreißlaschen versehen sein, die so angeordnet sind, dass sie beim Bewegen der Kartusche in der Halterung ein oder mehrere Austrittsöffnungen freigeben. Die Aufreißlaschen können beispielsweise auf der Oberseite der Kartusche so angeordnet sein, dass sie beim Bewegen in der Kartusche in der Halterung umknicken und ein oder mehrere Öffnungen auf der Oberseite der Kartusche erzeugen, die mit der Entmischungskammer der Kartusche kommunizieren.

Die Quelle des Trägergases kann beispielsweise einen Kompressor oder eine Druckgasflasche, die vorzugsweise ein inertes Gas enthält, umfassen.

Gemäß einer bevorzugten Ausführungsform umfasst der Aerosolerzeuger außerdem eine Spannungsquelle, die mit der Kartusche beispielsweise in Form einer in die Kartusche eintreibbaren Elektrode verbindbar ist.

Die Erfindung könnte zum Beispiel in einer Nebelbadvorrichtung mit einem Bade- oder Behandlungsraum zur Aufnahme von mindestens einem zu behandelnden Lebewesen oder von Körperteilen eines zu behandelnden Lebewesens, wobei die Nebelbadvorrichtung einen Aerosolerzeuger der oben beschriebenen Art umfassen könnte. Bei der Nebelbadvorrichtung kann es sich beispielsweise um eine Aerosolkabine handeln, wie sie in den internationalen Patentanmeldungen WO 00/44331 A1 und WO 2009/087053 A1 beschrieben worden ist.

Bevorzugt weist die Nebelbadvorrichtung außerdem Mittel auf, um das zu behandelnde Lebewesen oder dessen in der Nebelbadvorrichtung befindlichen Körperteile zu erden oder elektrostatisch gegenpolig zu der zu zerstäubenden Flüssigkeit aufzuladen. Außerdem kann durch ein von außen angelegtes elektromagnetisches Feld kann die Anlagerung der geladenen Nanoteilchen an den Körper oder Körperteile des Lebewesens erhöht werden.

Die typischen, im Rahmen einer Behandlung zerstäubten Flüssigkeitsmengen hängen von den Abmessungen des Behandlungsraums, in welchem der Aerosolnebel erzeugt werden soll, ab. Für Teilkörperbehandlungen einer Person reichen Flüssigkeitsmengen von wenigen Millilitern bereits aus. Für die Behandlung einer Person in einer die gesamte Person aufnehmenden Nebelbadvorrichtung werden typischerweise 1 bis 25, vorzugsweise 1-20 ml Flüssigkeit zerstäubt, während bei der Behandlung größerer Tiere, wie beispielsweise Pferden in entsprechenden Stallungen oder Kammern, bis zu einigen 100 ml Flüssigkeit pro Behandlung zerstäubt werden können. Je nach Menge der zu zerstäubenden Flüssigkeit wird die erfindungsgemäße Kartusche dimensioniert. Alternativ kann die erforderliche Flüssigkeitsmenge mittels mehrerer Kartuschen, die nacheinander oder gleichzeitig verwendet werden, zerstäubt werden. Das Prinzip der erfindungsgemäßen Kartusche ist jedoch nicht an ein bestimmtes Volumen gebunden. Es sind auch wesentliche größere Fluidvolumina als die oben genannten denkbar. So können beispielsweise bei der Luftdesinfektion größere Räume über einen längeren Zeitraum, etwa einer Flugzeugkabine während eines Interkontinentalflugs, deutlich größere Kartuschen eingesetzt werden. Es sind auch Varianten denkbar bei denen die erfindungsgemäße Kartusche nach- oder wiederbefüllbar ausgestaltet ist. Die Kartusche kann dazu beispielsweise eine Nachfüllöffnung aufweisen oder über eine Nachfüllleitung mit einem Vorratsgefäß für das zu zerstäubende Fluid verbunden sein.

Die Erfindung wird im Folgenden anhand eines in der beigefügten Zeichnung dargestellten Ausführungsbeispiels näher erläutert. In der Zeichnung zeigt:
- Fig. 1: eine Nebelbadvorrichtung, die zur Durchführung des erfindungsgemäßen Verfahrens geeignet ist;
- Fig. 2: eine Prinzipskizze einer Ausführungsform des erfindungsgemäßen Aerosolerzeugers;
- Fig. 3: eine bevorzugte Ausführungsform der erfindungsgemäßen Kartusche in der Seitenansicht;
- Fig. 4: eine Unteransicht der Kartusche der Fig. 3;
- Fig. 5: eine Draufsicht auf die Kartusche der Fig. 3;
- Fig. 6: einen axialen Längsschnitt durch die Kartusche der Fig. 3 entlang der Linie VI-VI der Fig. 5;
- Fig. 7: eine Detailvergrößerung des Querschnitts der Fig. 6;
- Fig. 8: einen axialen Längsschnitt der Kartusche der Fig. 3 entlang der Linie VIII-VIII der Fig. 5;
- Fig. 9: eine vergrößerte Detailansicht des Querschnitts der Fig. 8;
- Fig. 10: eine Variante des Oberteils der Kartusche der Fig. 3;
- Fig. 11: eine Draufsicht auf die Variante des Oberteils der Fig. 10;
- Fig. 12: eine erste Variante der Aufreißlasche der Fig. 10;
- Fig. 13: eine zweite Variante der Aufreißlasche der Fig. 10; und
- Fig. 14: eine Variante des Antriebs der Kartuschenaufnahme der Fig. 2.

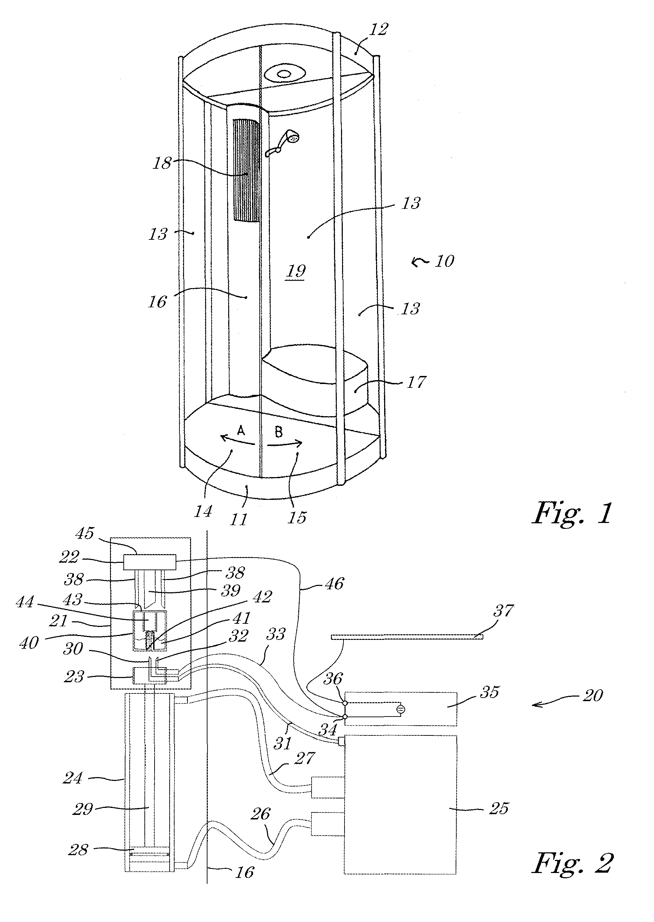

Fig. 1 zeigt eine insgesamt mit der Bezugsziffer 10 bezeichnete Nebelbadvorrichtung, wie sie beispielsweise schon in den internationalen Patentanmeldungen WO 00/44331 A1 und WO 2009/087053 A1 beschrieben wurde, die nicht Teil del Erfindung ist.

Die Nebelbadvorrichtung 10 wird von einer Bodenplatte 11, einer Deckenplatte 12, Seitenscheiben 13 aus Acrylglas und zwei entlang der Pfeile A, B beweglichen Vorderscheiben 14, 15, die den Zugang zu der Behandlungskammer 10 ermöglichen, begrenzt. In der Nebelbadvorrichtung 10 erstreckt sich zwischen der Bodenplatte 11 und der Deckenplatte 12 ein Rohr 16. Zudem ist an einer Oberseite der Bodenplatte 11 eine wasserdichte Sitzbank 17 angeordnet. Anstelle der in WO 00/44331 A1 und WO 2009/087053 A1 beschriebenen Mittel zur Aerosolerzeugung ist in der Nebelbadvorrichtung 10 in dem Rohr 16 ein im Folgenden näher beschriebener erfindungsgemäßer Aerosolerzeuger angeordnet. Das erzeugte Aerosol kann über ein in dem Rohr 16 angeordnete Gitter 18 in den Behandlungsraum 19 strömen.

Falls, wie im Folgenden näher beschrieben wird, die erzeugten Aerosolpartikel elektrostatisch aufgeladen sind, kann die Sitzbank 17 geerdet oder gegenpolig geladen werden. Wenn die zu behandelnde Person auf der Sitzbank Platz nimmt, werden die im Behandlungsraum 19 befindlichen Aerosolteilchen von der zu behandelnden Person angezogen. Entsprechend können beispielsweise die Seitenwände 13 der Nebelbadvorrichtung 10 aus einem leitfähigen Material bestehen, das nach Abschluss der Behandlung gegenpolig zu den Aerosolteilchen geladen wird. Die restlichen, im Behandlungsraum befindlichen Aerosolteilchen werden dann von den Seitenwänden angezogen und können anschließend leicht abgewischt werden. Alternativ oder zusätzlich kann ein (in Fig. 1 nicht dargestelltes) Sauggebläse vorgesehen sein, welches restliche Aerosolteilchen nach der Behandlung aus dem Behandlungsraum 19 absaugt. Auf diese Weise ist gewährleistet, dass auch die mit dem erfindungsgemäßen Aerosolerzeuger produzierten Aerosole im Sub-Mikrometerbereich vor einem weiteren Gebrauch der Nebelbadvorrichtung effektiv aus dem Behandlungsraum 19 entfernt werden.

In Fig. 2 ist ein insgesamt mit der Bezugsziffer 20 bezeichneter erfindungsgemäßer Aerosolerzeuger schematisch dargestellt, wie er in der Nebelbadvorrichtung 10 der Fig. 1 verwendet werden kann. Der Aerosolerzeuger 20 weist eine Halterung 21 für eine auswechselbare Kartusche auf, in welcher sich das zu zerstäubende Fluid befindet. Im dargestellten Beispiel weist die Halterung 21 ein festes Oberteil 22 und ein bewegliches Unterteil 23 auf, das mit einem Pneumatikantrieb 24 verbunden ist. Die Halterung 21 und der Pneumatikantrieb 24 können beispielsweise in dem Rohr 16 der Nebelbadvorrichtung 10 der Fig. 1 angeordnet sein. Der Pneumatikantrieb 24 ist mit einem Kompressor 25 verbunden, der beispielsweise unter der Sitzbank 17 der Nebelbadvorrichtung 10 angeordnet werden kann. Zwei abwechselnd mit Druckluft beaufschlagbare Leitungen 26, 27 führen von dem Kompressor zum Pneumatikantrieb, um einen Kolben 28 auf und ab zu bewegen. Der Kolben 28 ist über einen Kolbenschaft 29 mit dem beweglichen Unterteil 23 der Halterung 21 verbunden. In der Halterung 23 ist ein zentraler, hohler Dorn 30 angeordnet, der über eine Leitung 31 ebenfalls mit dem Kompressor 25 verbunden ist und mit Druckluft beaufschlagt werden kann. Ferner ist im beweglichen Unterteil 23 der Halterung 21 eine Metallspitze 32 angeordnet, die über eine elektrische Leitung 33 mit einem Pol 34 einer Niederstromquelle 35 verbunden ist. Der andere Pol 36 der Niederstromquelle 35 kann beispielsweise mit einer in die Sitzfläche 17 der Nebelbadvorrichtung 10 der Fig. 1 eingelassenen Metallplatte 37 verbunden werden. Das feste Oberteil 22 der Halterung 21 weist in der dargestellten Ausführungsform einen zentralen Führungsdorn 38 und kreisförmig um den Führungsdorn 38 herum angeordnete hohle Dorne 39 auf.

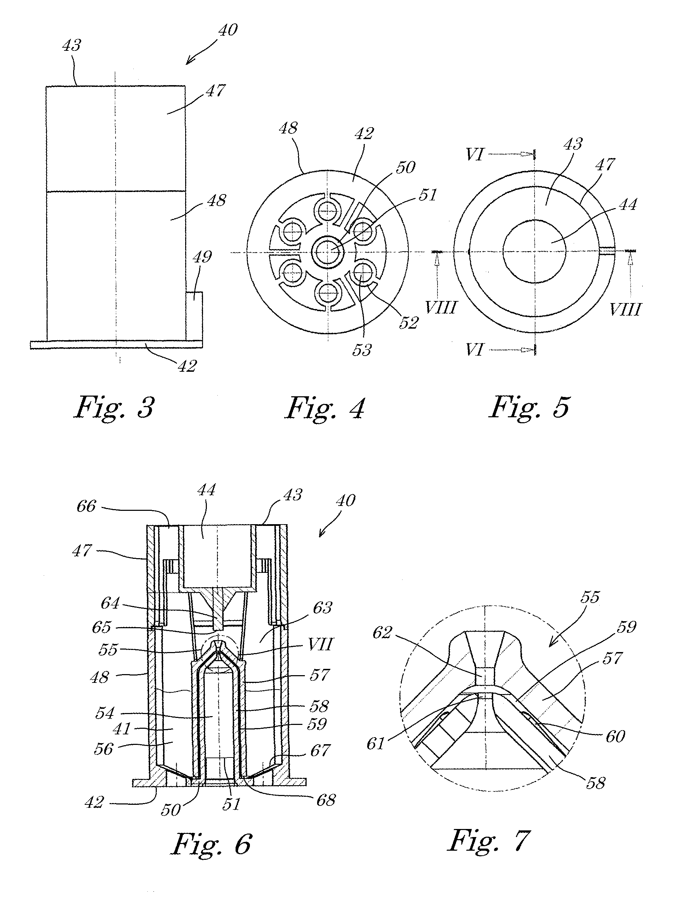

In die Halterung 21 wird eine zunächst hermetisch verschlossene Kartusche 40 eingesetzt, die weiter unten im Zusammenhang mit den Fig. 3-13 detaillierter beschrieben wird. In der Kartusche 40 befindet sich das zu zerstäubende Fluid 41. Über den Pneumatikantrieb 24 wird die Kartusche 40 so zwischen Oberteil 22 und Unterteil 23 der Halterung 21 zusammengepresst, dass der hohle Dorn 30 und die Metallspitze 32 in die Unterseite 42 der Kartusche 40 eindringen können, während die Dorne 39 die Oberseite 43 der Kartusche durchbohren. Der Führungsdorn 38 greift in diesem Fall in eine in der Oberseite der Kartusche 40 vorgesehene Vertiefung 44 ein. Zur Erzeugung des Nanoaerosols wird Druckluft über den hohlen Dorn 30 in die Kartusche 40 eingeblasen. Die Druckluft und kleinere Aerosolpartikel im Sub-Mikrometerbereich verlassen die Kartusche 40 dann über die hohlen Dorne 39 und treten aus der Oberseite 45 des Oberteils 22 der Halterung 21 in die Umgebung aus. Um ein Ablagern von Aerosolpartikeln an dem Oberteil 22 der Halterung 21 zu vermeiden, kann das Oberteil 22 über eine Leitung 46 mit dem gleichen Pol 34 der Spannungsquelle 35 verbunden werden, mit dem auch die als Elektrode dienende Metallspitze 32 verbunden ist, so dass das Oberteil 22 und die erzeugten Aerosolpartikel gleichnamig aufgeladen werden.

In Fig. 3 ist eine erste Variante der erfindungsgemäßen Kartusche 40 in einer schematischen Seitenansicht dargestellt. Das Außengehäuse der Kartusche 40 besteht aus zwei Spritzgussteilen 47, 48, die nach Einfüllen des zu zerstäubenden Fluids für den Benutzer unlösbar verbunden werden, beispielsweise durch Verkleben oder durch Ultraschall-Verschweißen. In dem in Fig. 3 dargestellten versiegelten Zustand ist das zu zerstäubende Fluid hermetisch dicht in der Kartusche 40 verschlossen. Das Unterteil 48 der Kartusche 40 weist eine vorspringende Nase 49 als Positionierhilfe zum korrekten Einsetzen der Kartusche in die Halterung 21 des Aerosolerzeugers der Fig. 2 auf. Im dargstellten Beispiel besitzt die Kartusche eine Höhe von etwa 55 mm und einen Durchmesser von etwa 30 mm. Mit einer solchen Kartusche können einige Milliliter eines Fluids in der Nebelbadvorrichtung der Figur 1 abgegeben werden.

In Fig. 4 ist die Unterseite 42 der Kartusche 40 der Fig. 3 dargestellt. Die Unterseite 42 weist eine zentrale Öffnung auf, in die ein Stopfen 50 eingesetzt ist. Der hohle Dorn 41 kann in den Stopfen eingreifen und dessen Boden 51 durchbohren und so ins Innere der Kartusche 40 eindringen. Ferner sind mehrere Ringe 52 ausgeformt, deren Böden 53 ebenfalls verschlossen sind. Einer der Böden 53 der Ringe 52 kann beispielsweise von dem als Elektrode dienenden Dorn 32 durchstoßen werden.

Fig. 5 zeigt eine Draufsicht auf die Kartusche 40 der Fig. 3. Man erkennt, dass die Oberseite 43 des Oberteils 47 der Kartusche 40 als Ring ausgebildet ist, welcher die Vertiefung 44 begrenzt, in welche der Führungsdorn 38 der Halterung 21 des Aerosolerzeugers 20 eingreifen kann. Die hohlen Dorne 39 der Halterung 21 sind vorzugsweise so angeordnet, dass beim Eindringen der Dorne in die Oberseite 43 gleichmäßig auf dem Ring 43 verteilte Öffnungen in die Oberseite der Kartusche 40 eingebracht werden.

Fig. 6 zeigt einen Längsschnitt der Kartusche 40 der Fig. 3 entlang der durch die Linie VI-VI in Fig. 5 definierten Ebene. Wie man erkennt, weist die Kartusche eine zentrale Bohrung 54 auf, welche vom Boden 51 des zentralen Rings 50 ausgeht und in eine Düsespitze 55 mündet. In Fig. 7 ist die in Fig. 6 mit dem Kreis VII begrenzte Düsenspitze 55 vergrößert dargestellt.

Die Bohrung 54 dient als Zuleitung für das Trägergas, welches in die Düse 55 eingeblasen wird. Die Düsenspitze 55 ist als Venturi-Düse ausgebildet, so dass das die Düse durchströmende Trägergas das zu zerstäubende Fluid aus einem Reservoir 56 der Kartusche 40 ansaugen kann. Die Düse 55 besteht dazu aus einem Außenmantel 57, der im vorliegenden Fall zusammen mit dem Unterteil 48 als einstückiges Spritzgussteil ausgebildet ist. Als separates Spritzgussteil wird bei der Herstellung der Kartusche ein Innenmantel 58 durch den Boden 42 der Kartusche in den Außenmantel so eingesetzt, dass zwischen Innenmantel und Außenmantel ein Ringkanal 59 ausgespart bleibt, der mit dem Reservoir 56 kommuniziert und über den das zu zerstäubende Fluid 41 in die Düsenspitze 55 gesaugt werden kann. Damit der Ringkanal 59 auch bei Druckbeaufschlagung der zentralen Bohrung 54 offen bleibt, können an der Oberseite des Innenmantels 58 oder an der Unterseite des Außenmantels 57 mehrere Rippen 60 als Abstandshalter vorgesehen sein (vgl. insbesondere Fig. 7). Das aus der Öffnung 61 des Innenmantels 58 durch die Öffnung 62 des Außenmantels 57 strömende Trägergas sorgt in dem Ringspalt 59 für einen Unterdruck, der das zu zerstäubende Fluid aus dem Reservoir 56 zur Öffnung 62 saugt. Die Öffnung 62 des Außenmantels 57 hat im dargestellten Beispiel einen Öffnungswinkel von etwa 32°. Der Durchmesser der Öffnung 61 beträgt im Beispiel der Fig. 3 bis 9 etwa 0,6 mm und der Durchmesser der Öffnung 62 an der engsten Stelle 0,9 mm. Wird die Zuleitung 54 mit einem Druck des Trägergases von ca. 2 Bar beaufschlagt, so entstehen bei Verlassen der Düsenspitze 55 Fluidpartikel im Sub-Mikrometerbereich und Fluidpartikel mit Durchmessern von mehr als 1 µm. Um die größeren von den kleineren Fluidpartikeln zu trennen, ist in der Kartusche im Übergangsbereich von Oberteil 47 und Unterteils 48 eine Entmischungskammer 63 vorgesehen. Die Entmischungskammer 43 umfasst eine Ablenkeinrichtung 64, die einen Stempel 65 umfasst, der im zusammengebauten Zustand der Kartusche etwa 2 mm oberhalb der Düsenöffnung 62 angeordnet ist. Das aus der Düse 62 austretende Gemisch aus größeren und kleineren Fluidpartikeln wird im Rahmen des vorgegebenen Öffnungswinkels der Düse 62 im Wesentlichen axial nach oben aus der Düse 62 ausgestoßen und trifft unmittelbar danach auf die Ablenkeinrichtung 64. Die Fluidpartikel werden entsprechend zur Seite abgelenkt, so dass größere Fluidpartikel unter der Einwirkung der Schwerkraft zurück ins Fluidreservoir 56 fließen können, während kleinere Fluidpartikel im Sub-Mikrometerbereich durch Öffnungen 66, welche durch die Dorne 39 der Halterung 21 in die Oberseite 43 der Kartusche 40 eingebracht werden, ausgetragen werden. Mit der erfindungsgemäßen Kartusche kann also gewährleistet werden, dass das in der Kartusche befindliche Fluid 41 im Wesentlichen vollständig in Form eines Nanoaerosols in die Umgebung abgegeben wird. Um auch letzte Reste des Fluids ausnützen zu können, weist der Boden 67 des Fluidreservoirs 56 eine zum Fußspalt 68 des Kanals 59 gewichtete Neigung auf.

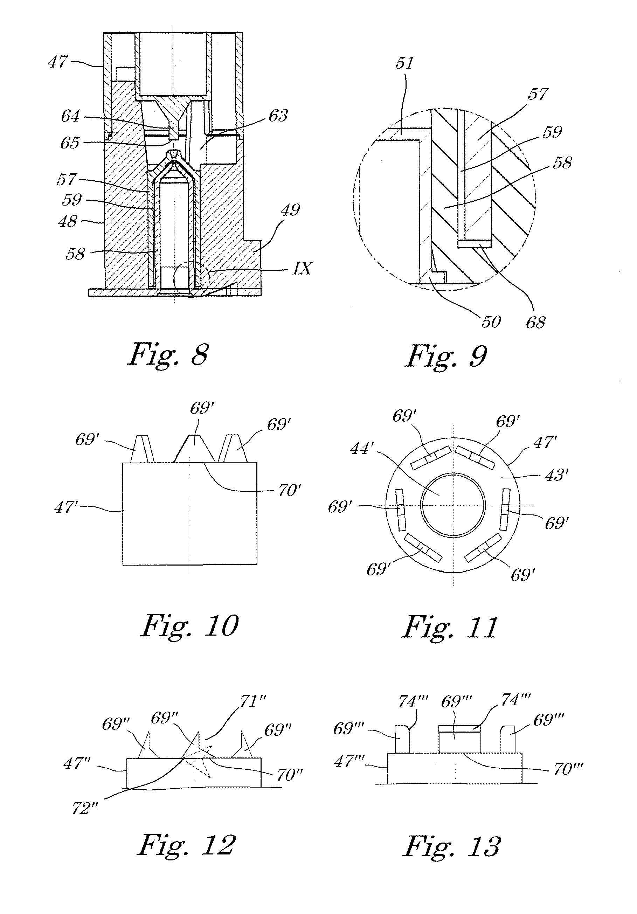

Fig. 8 zeigt einen Längsschnitt der Kartusche 40 der Fig. 3 entlang der durch die Linie VIII-VIII definierten Ebene. In dieser Ebene liegen nach innen gerichtete Verstärkungsrippen der Kartusche.

In Fig. 9 ist der in Fig. 8 durch den Kreis IX begrenzte Fußbereich der Innenhülse 58 und der Außenhülse 57 mit dem Fußspalt 68 des zwischen Innenhülse und Außenhülse gebildeten Kanals 59 detaillierter dargestellt.

Fig. 10 zeigt eine Variante 47' des Oberteils 47 der Kartusche 40 der Fig. 3. In diesem Fall weist das Oberteil 47' auf seiner Oberfläche 43' Aufreißlaschen 69' auf, die mit dem Oberteil 47' als einstückiges Bauteil ausgeformt sind. Wenn die Aufreißlaschen 69' umgeknickt werden, geben sie in ihrem Fußbereich 70' Öffnungen in der Oberseite 43' des Oberteils 47' frei, durch welche das Nanoaerosol aus der Kartusche entweichen kann. Um ein Umknicken der Aufreißlaschen 69' zu gewährleisten, weist das Oberteil 22 der Halterung 21 der Fig. 2 keine Dornen 39 auf, sondern kann beispielsweise eine leicht konische Innenfläche aufweisen, welche die Aufreißlaschen nach innen drückt, wenn der Pneumatikantrieb 24 über das bewegliche Unterteil 23 der Halterung 21 die Kartusche in das Oberteil 22 presst. Somit kann das Oberteil 22 der Haltung besonders einfach gereinigt werden.

Fig. 11 zeigt eine Draufsicht auf das Oberteil 47' der Fig. 10.

Fig. 12 zeigt eine Variante 69" der Aufreißlaschen 69' der Fig. 10. Im dargestellten Beispiel sind die Aufreißlaschen 69" von den dreieckigen Aufreißlaschen 69' der Fig. 10 abgeleitet, weisen aber auf einer Seite eine Aussparung 71" auf. Zur Ausbildung einer Öffnung 70" in der Oberseite des Oberteils 47" der Fig. 12 werden die Aufreißlaschen 69" nicht nach innen gebogen, sondern in der Ebene der Fig. 12 nach unten gedrückt. Die Aufreißlaschen 69" kippen dann im Wesentlichen um einen Eckpunkt 72", so dass die in Fig. 12 gestrichelt dargestellte Konfiguration der Aufreißlasche entsteht, wo eine Öffnung 70" in der Oberfläche 43" freigegeben wird.

Fig. 13 zeigt eine weitere Variante 69"' der Aufreißlasche 69' der Fig. 10. Die Aufreißlasche 69"' der Fig. 13 im Wesentlichen rechteckig ausgebildet und weist eine abgerundete Oberkante 74"' ist. Dadurch kann das Oberteil 22 der Halterung 21 (Fig. 2) als Hohlzylinder ausgebildet sein, was dessen Reinigung noch weiter erleichtert.

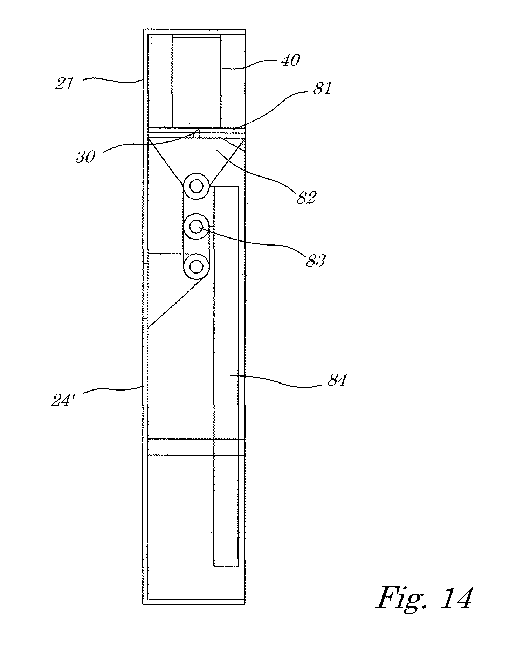

In Fig. 14 ist schließlich eine kostengünstigere Variante 24' des Kartuschenantriebs 24 des Aerosolerzeugers der Fig. 2 schematisch dargestellt. Der Aerosolerzeuger entspricht im wesentlichen dem Aerosolerzeuger 20 der Fig. 2 und ist der Übersichtlichkeit halber nicht mehr mit all seinen Komponenten dargestellt. Der Aerosolerzeuger weist wiederum eine Halterung 21 für eine auswechselbare Kartusche 40 auf, in welcher sich das zu zerstäubende Fluid befindet. Statt eines Pneumatikantriebs (Bezugsziffer 24 in Fig. 2) zum Eintreiben der Dornen in die Kartusche ist bei der Variante der Fig. 14 ein rein mechanischer Antrieb 24' vorgesehen. Die Kartusche 40 wird auf einen in der Halterung 21 beweglich montierten oberen Schlitten 81 gesetzt. Auf einem beweglichen unteren Schlitten 82 befindet sich der zentralen, hohlen Dorn 30, wie er bereits aus Fig. 2 bekannt ist. Im dargestellten Beispiel kann der untere Schlitten 82 mittel eines von Hand betätigbaren Kniehebels 83 nach oben bewegt werden, um einerseits den Dorn 30 in die Unterseite der Kartusche 40 einzustechen und andererseits den oberen Schlitten 81 mit der Kartusche 40 gegen das (in Fig. 14 nicht dargestellte) Oberteil 22 der Halterung zu drücken. Dazu weist der Kniehebel 83 eine vom Benutzer bedienbaren Betätigungsarm 84 auf. Das nicht dargestellte Oberteil 22 weist je nach Ausgestaltung der Kartusche 40 zur Erzeugung der Austrittsöffnungen in der Kartusche Dornen (vergleichbar den Dornen 38 in Fig. 2) oder nur einen geeigneten Gegenring zum Umknicken von Aufreißlaschen der Kartusche 40 auf. Der (in Fig. 14 nicht dargestellte) Kompressor 25 ist dann nicht mehr für den Antrieb, sondern nur noch für die Erzeugung von Druckluft zuständig, die durch den hohlen Dorn 30 in die Kartusche 40 eingeblasen wird. Selbstverständlich kann auch bei dieser Variante eine (hier nicht dargestellte) Elektrode (analog der Elektrode 32 der Fig. 2) in die Kartusche eingestochen werden, um das Fluid elektrostatisch aufzuladen.

## Patentansprüche

1. Verfahren zur Erzeugung eines Nanoaerosols, wobei man
in einer Düse wenigstens ein zu zerstäubendes Fluid durch eine Düsenöffnung der Düse entlang einer Austrittsrichtung in Form von Fluidpartikein zerstäubt,
die zerstäubten Fluidpartikel aus der Austrittsrichtung ablenkt und größere Fluidpartikel von kleineren Fluidpartikeln zumindest teilweise abtrennt,
die abgetrennten größeren Fluidpartikel in das zu zerstäubende Fluid zurückführt, und
die kleineren Fluidpartikel an die Umgebung abgibt,
wobei man eine Kartusche verwendet, in welcher die Düse und das zu zerstäubende Fluid angeordnet sind,
wobei man in der Düse einen Strom eines Trägergases erzeugt und das wenigstens eine zu zerstäubende Fluid in der Düse mit dem Trägergas in Kontakt bringt,
**dadurch gekennzeichnet, dass** man
eine hermetisch verschlossene Kartusche verwendet und vor Gebrauch Öffnungen für die Zufuhr des Trägergases und die Abgabe der zerstäubten kleineren Fluidpartikel an die Umgebung in die Kartusche einbringt.

2. Verfahren gemäß Anspruch 1, wobei man die abgetrennten größeren Fluidpartikel mittels Schwerkraft in das zu zerstäubende Fluid zurückführt, während man die kleineren Fluidpartikel in einer von der Wirkungsrichtung der Schwerkraft verschiedenen Richtung an die Umgebung abgibt.

3. Verfahren gemäß einem der Ansprüche 1 oder 2, wobei das zu zerstäubende Fluid zumindest einen Wirkstoff umfasst,
wobei der Wirkstoff ausgewählt ist aus der Gruppe von Wirkstoffen bestehend aus: Desinfektionsmitteln, desodorierenden Mitteln, Duftstoffen, kosmetischen Mitteln, diagnostischen und/oder therapeutischen Mitteln zur Behandlung von Lebewesen.

4. Verfahren gemäß einem der Ansprüche 1 bis 3, wobei man zumindest einen Teil der abzugebenden kleineren Fluidpartikel elektrostatisch auflädt.

5. Kartusche zur Erzeugung eines Nanoaerosols, mit
wenigstens einem Reservoir für das zu zerstäubende Fluid,
einer Entmischungskammer, die eine Ablenkeinrichtung und wenigstens eine Austrittsöffnung für das zu zerstäubende Fluid aufweist,
einer Düse, die wenigstens eine in die Entmischungskammer mündende Düsenöffnung aufweist, und
wenigstens einen von dem Reservoir ausgehenden Kanal, der in die Düse mündet,
wobei wenigstens eine in die Düse mündende Zuleitung für ein Trägergas vorgesehen ist,
**dadurch gekennzeichnet,**
**dass** die Zuleitung für das Trägergas als zentrale Bohrung ausgebildet ist, die an einem Ende in den Boden der Kartusche mündet und an ihrem anderen Ende in die Düse übergeht, und
**dass** die Kartusche hermetisch verschlossen ist und
die in den Boden der Kartusche mündende Bohrung durch eine durchstossbare Wand verschlossen ist.

6. Kartusche gemäß Anspruch 5, **dadurch gekennzeichnet, dass** eine kommunizierende Verbindung zwischen dem Reservoir und der Entmischungskammer vorgesehen ist.

7. Kartusche gemäß einem der Ansprüche 5 oder 6, **dadurch gekennzeichnet, dass** die wenigstens eine Austrittsöffnung für das zu zerstäubende Fluid durch eine durchstossbare oder aufreißbare Wand verschlossen ist.

8. Kartusche gemäß einem der Ansprüche 5 bis 7, **dadurch gekennzeichnet, dass** die Düse als Venturidüse ausgebildet ist.

9. Kartusche gemäß einem der Ansprüche 5 bis 8, **dadurch gekennzeichnet, dass** Mittel zu Verbindung des Reservoirs mit einer elektrischen Spannungsquelle vorgesehen sind.

10. Kartusche gemäß einem der Ansprüche 5 bis 9, **dadurch gekennzeichnet, dass** die Kartusche aus zumindest zwei Bauteilen besteht, die nach Einfüllen des zu zerstäubenden Fluids miteinander verbindbar sind.

11. Kartusche gemäß Anspruch 10, **dadurch gekennzeichnet, dass** die beiden Bauteile unlösbar miteinander verbunden sind.

12. Kartusche gemäß einem der Ansprüche 5 bis 11, **dadurch gekennzeichnet, dass** sich in dem Reservoir ein zu zerstäubendes Fluid befindet.

13. Aerosolerzeuger, der eine Halterung zur Aufnahme wenigstens einer Kartusche nach Anspruch 12 und Mitteln zum Ausstoßen des in der Kartusche enthaltenden Fluids umfaßt.

14. Aerosolerzeuger gemäß Anspruch 13, **dadurch gekennzeichnet, dass** die Mittel zum Ausstoßen des in der Kartusche enthaltenen Fluids eine Quelle eines Trägergases aufweisen, wobei die Halterung wenigstens einen ersten hohlen Dorn, der mit der Quelle eines Trägergases kommuniziert und in die Zuleitung für das Trägergas der Kartusche eingreifen kann, und wenigstens einen zweiten hohlen Dorn, der mit der Umgebung kommuniziert und in die Austrittsöffnung für das zu zerstäubende Fluid der Kartusche kann, umfasst.

15. Aerosolerzeuger gemäß Anspruch 14, **dadurch gekennzeichnet, dass** die Quelle eines Trägergases einen Kompressor oder eine Druckgasflasche, die ein inertes Gas enthält, umfasst, und dass außerdem eine Spannungsquelle vorgesehen ist, die mit der Kartusche verbindbar ist.

## Claims

1. Method for generating a nano-aerosol, wherein
in a nozzle at least one fluid, which is for being sprayed, is sprayed through a nozzle orifice of the nozzle in form of fluid particles along an outlet direction,
the sprayed fluid particles are deflected out of the outlet direction and larger fluid particles are at least partially separated from smaller fluid particles,
the separated larger fluid particles are returned into the fluid, which is for being sprayed, and
the smaller fluid particles are released to the surrounding,
wherein a cartridge is used, in which the nozzle and the fluid, which is for being sprayed, are arranged,
wherein a flow of a carrier gas is generated in the nozzle and the at least one fluid, which is for being sprayed, is brought into contact with the carrier gas in the nozzle,
**characterized in that**
a hermetically closed cartridge is used and
orifices, for the supply of the carrier gas and the release of the sprayed smaller fluid particles to the surrounding, are formed before usage.

2. Method according to claim 1, wherein the separated larger fluid particles are returned into the fluid, which is for being sprayed, by means of gravity force, while the smaller fluid particles are released to the surroundings in a direction different from the direction of action of the gravity force.

3. Method according to one of the claims 1 or 2, wherein the fluid, which is for being sprayed, comprises at least one active ingredient,
wherein the active ingredient is selected from the group of active ingredients consisting of disinfectants, deodorants, fragrances, cosmetics, diagnostic and/or therapeutic agent for treating organisms.

4. Method according to one of the claims 1 to 3, wherein at least one part of the smaller fluid particles, which are for being released, are electrostatically charged.

5. Cartridge for generating a nano-aerosol, comprising
at least one reservoir for the fluid, which is for being sprayed,
a separation chamber, comprising a deflection means and at least one outlet orifice for the fluid, which is for being sprayed,
a nozzle, comprising at least one nozzle orifice leading into the separation chamber, and
at least one channel extending from the reservoir, which channel leads into the nozzle,
wherein at least one supply for a carrier gas is provided, which supply leads into the nozzle,
**characterized in that**
the supply for the carrier gas is formed as a central bore
which leads at one end into the bottom of the cartridge and
at its other end passes into the nozzle,
and **in that**
the cartridge is hermetically closed and
the bore, which leads into the bottom of the cartridge, is closed by means of a pierceable wall.

6. Cartridge according to claim 5, **characterized in that** a communicating connection between the reservoir and the separation chamber is provided.

7. Cartridge according to one of the claims 5 or 6, **characterized in that** the at least one outlet orifice for the fluid, which is for being sprayed, is closed by a pierceable or tearable wall.

8. Cartridge according to one of the claims 5 to 7, **characterized in that** the nozzle is formed as a Venturi nozzle.

9. Cartridge according to one of the claims 5 to 8, **characterized in that** means are provided for connecting the reservoir with an electric voltage source.

10. Cartridge according to one of the claims 5 to 9, **characterized in that** the cartridge consists of at least two components, which are connectable to each other after filling in the fluid, which is for being sprayed.

11. Cartridge according to claims 10, **characterized in that** both of the components are connected to each other in an unreleasable manner.

12. Cartridge according to one of the claims 5 to 11, **characterized in that** a fluid, which is for being sprayed, is located within the reservoir.

13. Aerosol generator, comprising
a holding means for accommodation of at least one cartridge according to claim 12, and
means for ejecting the fluid, which is contained in the cartridge.

14. Aerosol generator according to claim 13, **characterized in that** the means for ejecting the fluid, which is contained in the cartridge, comprise a carrier gas,
wherein the holding means comprises
at least one first hollow pin which communicates with the source of a carrier gas and which pin can engage with the supply for the carrier gas of the cartridge, and at least one second hollow pin, which communicates with the surrounding and which can into the outlet orifice of the cartridge, for the fluid, which is for being sprayed.

15. Aerosol generator according to claim 14, **characterized in that** the source of a carrier gas comprises a compressor or a compressed gas bottle, which comprises an inert gas, and further **in that**
a voltage source is intended, which is connectable with the cartridge.

## Revendications

1. Procédé de génération d'un nanoaérosol, sachant qu'on
pulvérise dans une buse au moins un fluide à pulvériser par une ouverture de la buse le long d'un sens de sortie sous la forme de particules de fluide,
dévie les particules de fluide pulvérisées du sens de sortie et sépare au moins en partie des particules de fluide plus grandes des particules de fluide plus petites,
ramène les particules de fluide plus grandes séparées dans le fluide à pulvériser, et
libère les particules de fluide plus petites dans l'environnement,
sachant qu'on utilise une cartouche, dans laquelle la buse et le fluide à pulvériser sont agencés,
sachant qu'on génère dans la buse un courant d'un gaz porteur et amène en contact l'au moins un fluide à pulvériser dans la buse avec le gaz porteur
**caractérisé en ce qu'**on
utilise une cartouche fermée hermétiquement et introduit avant l'utilisation des ouvertures pour l'amenée du gaz porteur et la libération des particules de fluide plus petites pulvérisées dans l'environnement dans la cartouche.

2. Procédé selon la revendication 1, sachant qu'on ramène les particules de fluide plus grandes séparées par la force de gravité dans le fluide à pulvériser alors qu'on libère les particules de fluide plus petites dans un sens différent du sens d'action de la force de gravité dans l'environnement.

3. Procédé selon l'une quelconque des revendications 1 ou 2, sachant que le fluide à pulvériser comporte au moins une substance active, sachant que la substance active est sélectionnée dans le groupe de substances actives se composant des : agents désinfectants, agents désodorisants, parfums, agents cosmétiques, agents diagnostiques et/ou thérapeutiques pour le traitement d'êtres vivants.

4. Procédé selon l'une quelconque des revendications 1 à 3, sachant qu'on charge électrostatiquement au moins une partie des particules de fluide plus petites à libérer.

5. Cartouche pour la génération d'un nanoaérosol avec
au moins un réservoir pour le fluide à pulvériser, une chambre de séparation qui présente un dispositif de déviation et au moins une ouverture de sortie pour le fluide à pulvériser,
une buse qui présente au moins une ouverture de buse débouchant dans la chambre de séparation, et
au moins un canal sortant du réservoir qui débouche dans la buse,
sachant qu'au moins une conduite d'amenée débouchant dans la buse est prévue pour un gaz porteur,
**caractérisée en ce que**
la conduite d'amenée pour le gaz porteur est réalisée comme un perçage central qui débouche sur une extrémité dans le fond de la cartouche et passe sur son autre extrémité dans la buse, et
**en ce que** la cartouche est fermée hermétiquement et le perçage débouchant dans le fond de la cartouche est fermé par une paroi transperçable.

6. Cartouche selon la revendication 5, **caractérisée en ce qu'**une liaison communicante est prévue entre le réservoir et la chambre de séparation.

7. Cartouche selon l'une quelconque des revendications 5 ou 6, **caractérisée en ce que** l'au moins une ouverture de sortie pour le fluide à pulvériser est fermée par une paroi transperçable ou déchirable.

8. Cartouche selon l'une quelconque des revendications 5 à 7, **caractérisée en ce que** la buse est réalisée comme une buse Venturi.

9. Cartouche selon l'une quelconque des revendications 5 à 8, **caractérisée en ce que** des moyens sont prévus pour la liaison du réservoir à une source de tension électrique.

10. Cartouche selon l'une quelconque des revendications 5 à 9, **caractérisée en ce que** la cartouche se compose au moins de deux éléments qui peuvent être reliés entre eux après le remplissage du fluide à pulvériser.

11. Cartouche selon la revendication 10, **caractérisée en ce que** les deux éléments sont reliés entre eux de manière indétachable.

12. Cartouche selon l'une quelconque des revendications 5 à 11, **caractérisée en ce qu'**un fluide à pulvériser se trouve dans le réservoir.

13. Générateur d'aérosol qui comporte un support pour le logement au moins d'une cartouche selon la revendication 12 et des moyens d'éjection du fluide contenu dans la cartouche.

14. Générateur d'aérosol selon la revendication 13, **caractérisé en ce que** les moyens d'éjection du fluide contenu dans la cartouche présentent une source d'un gaz porteur, sachant que le support comporte au moins une première épine creuse qui communique avec la source d'un gaz porteur et peut s'engager dans la conduite d'amenée pour le gaz porteur de la cartouche et au moins une seconde épine creuse qui communique avec l'environnement et peut s'engager dans l'ouverture de sortie pour le fluide à pulvériser de la cartouche.

15. Générateur d'aérosol selon la revendication 14, **caractérisé en ce que** la source d'un gaz porteur comporte un compresseur ou une bouteille de gaz comprimé qui contient un gaz inerte et **en ce qu'**une source de tension est de plus prévue, laquelle peut être reliée à la cartouche.
